# FASCICULE DE BREVET EUROPEEN

(11) **EP 0 663 205 B1**
(45) Date de publication et mention de la délivrance du brevet: **18.06.1997**
(21) Numéro de dépôt: 94402620.2
(22) Date de dépôt: 17.11.1994
(51) Int. Cl.: A61K 7/135

(54) **Compositions cosmétiques pour la décoloration des cheveux, procédé de synthèse et utilisation**
Kosmetische Zusammensetzungen für Haarbleichung, Syntheseverfahren und Anwendungen
Cosmetic compositions for hair bleaching, process for their synthesis and their use

(30) Priorité: 14.01.1994 FR 9400366
(43) Date de publication de la demande: 19.07.1995
(73) Titulaire: L'OREAL, 75008 Paris (FR)
(72) Inventeur: Tricaud, Caroline, F-95240 Cormeilles en Parisis (FR); Millequant, Jean, F-94100 Saint-Maur (FR); Sebag, Henri, F-75016 Paris (FR)
(74) Mandataire: Andral, Christophe André Louis

(56) Documents cités:
- EP-A- 0 574 696
- DE-A- 3 434 468
- FR-A- 2 276 809
- US-A- 4 327 751

## Description

La présente invention concerne des compositions cosmétiques décolorantes pour cheveux possédant des propriétés améliorées. Plus particulièrement encore, elle concerne des compositions de décoloration se présentant à l'état de poudres fines, anhydres, fluides, homogènes et non poussiérantes.

Il est connu de décolorer les cheveux à l'aide de pâtes (ou cataplasmes) appliquées directement sur les cheveux et obtenues en mélangeant, au moment de l'emploi, une composition décolorante à base de dérivés peroxydés (agents oxydants) avec de l'eau, ou plus habituellement encore avec de l'eau oxygénée. La composition décolorante est, de façon connue, constituée d'un dérivé peroxydé (composant essentiel), généralement un persulfate ou un perborate de sodium, de potassium ou d'ammonium et parfois un sel d'acide percarboxylique ou un peroxyde, par exemple de baryum, de strontium, d'urée ou de mélamine. Le plus souvent, ces compositions contiennent en outre, de façon connue, des agents fortement alcalins tels que des métasilicates, des phosphates ou des carbonates alcalins ou alcalino-terreux (régulateurs de pH). Enfin, elles peuvent par ailleurs éventuellement contenir d'autres additifs ou adjuvents classiques dans le domaine : agents de controle du dégagement d'oxygène lors du mélange avec l'eau oxygénée, tels que carbonate de magnésium ou la magnésie; des épaississants, tels que des dérivés de cellulose (carboxymétnylcellulosse par exemple) ou l'amidon et ses dérivés, ou bien encore les gommes de guar, de xanthane et les alginates; des agents tensio-actifs, en particulier anioniques (alkylsulfates notamment); des colorants; des sequestrants; des parfums. De telles compositions décolorantes sont décrites, par exemple, dans "The Science of Hair Care" par C. ZVIAK, Marcel Decker Inc. 1986, pp 225-226.

Dans le brevet US-A-3 639 574, on a décrit des gels à base de peroxyde d'hydrogène, qu'on applique directement sur les cheveux pour les décolorer, et qui sont caractérisés par le fait qu'ils contiennent :
i) 1 à 20% en poids de peroxyde d'hydrogène,
ii) 20 à 77% en poids d'eau et,
iii 22 à 79% en poids d'un copolymère bloc polyoxyéthylène / polyoxypropylène bien sélectionné en motifs hydrophobe (C₃H₆O) et en motifs hydrophile (C₂H₄O) pour pouvoir former un gel, et de formule :

   HO(C₂H₄O)_{**b**} (C₃H₆O)_{**a**} (C₂H₄O)_{**b**}H

   dans laquelle,
   **a** est tel, que la partie hydrophobe représentée par (C₃H₆O), ait un poids moléculaire d'au moins 2250, (2250 à 4000) et que,
   **b** est tel, que la partie hydrophile représentée par (C₂H₄O), constitue de 10 à 90% en poids du copolymère.
      Ce document ne concerne pas des poudres décolorantes conformes à la présente invention.

Dans le brevet US-A-4 327 751, on a préparé des compositions décolorantes pour les cheveux, d'une grande souplesse d'emploi car elles permetttent de imiter les niveaux de décoloration, grâce à l'addition, dans un système décolorant oxydant alcalin classique et aqueux, d'une polyoléfine (polyéthylène - polypropylène - polybutène - polypentène - polyhexène), inerte vis-à-vis des systèmes oxydants alcalins, finement divisée (taille des particules 44 à 177 µ), et insoluble dans l'eau.

Les compositions décolorantes pour cheveux les plus couramment utilisées à ce jour se présentent sous forme de poudres (mélanges) de faible granulométrie, c'est à dire avec des particules dont la taille est généralement inférieure au millimètre, de préférence inférieure à quelques centaines de microns, ce qui permet une dissolution et/ou une dispersion facile et rapide dans l'eau oxygénée.

Toutefois, de telles compositions pulvérulentes, compte tenu de l'état finement divisé dans lequel elles se trouvent, présentent plusieurs inconvénients : elles sont fortement volatiles et émettent ainsi, lors de leur manipulation, des poussières nocives contenant des dérivés peroxydés et fortement irritantes pour les poumons; ces poudres sont par ailleurs délicates non seulement à manipuler mais également à doser (problème de poussièrage et de coulabilité).

Pour tenter de résoudre les problèmes susmentionnés, on a décrit dans la demande de brevet EP-A-0 560 088 une poudre décolorante pour cheveux totalement dépourvue de poussière (ou fines) et obtenue par ajout d'une huile ou d'une cire liquide au mélange de poudres initial (peroxyde solide + support solide à base des sels alcalins et des divers adjuvents décrits ci-avant).
Cependant, la poudre décolorante ci-dessus, bien qu'effectivement plus dense et moins poussiérante que les poudres décolorantes classiques, s'avère présenter d'autres désavantages, en particulier dus à la présence de l'huile ou de la cire, qui peuvent limiter fortement l'intéret de son utilisation. Ainsi, avec cette poudre, on constate que les mélanges avec l'eau oxygénée sont longs à réaliser et donnent des cataplasmes brillants et d'aspect huileux; qu'une élimination poussée du produit des cheveux est longue et fastidieuse; que les shampooings utilisés pour faciliter cette élimination après les décolorations, ne moussent plus; et que finalement, après l'opération de décoloration, les cheveux gardent un toucher gras et lourd, désagréable.

Or, à la suite d'importantes recherches menées sur la question, il a maintenant été trouvé par la Demanderesse qu'il est possible d'obtenir sous la forme de poudres des compositions cosmétiques décolorantes pour cheveux ne présentant aucun des effets indésirables précités attachés aux compositions de l'art antérieur, en introduisant dans ces dernières un polymère spécifique.

Cette découverte est la base de la présente invention.

Ainsi, selon la présente invention, il est maintenant proposé de nouvelles compositions cosmétiques anhydres pulvérulentes pour la décoloration des cheveux, à base de dérivés peroxydés comme agents oxydants, et caractérisées par le fait qu'elles comprennent en outre au moins un copolymère linéaire séquencé bloc et/ou statistique de type polyoxyéthylène/polyoxypropylène qui est à la fois anhydre et, à température ambiante, liquide d'une part et soluble dans l'eau d'autre part.

Le procédé de synthèse des poudres décolorantes selon l'invention, lui-même constitutif d'un second objet de la présente invention, consiste à mélanger, en milieu non solvant et à température ambiante, une poudre sèche décolorante classique à base de dérivés peroxydés solides, avec un copolymère tel que défini ci-dessus.

Un objet de l'invention est également l'utilisation des nouvelles compositions cosmétiques anhydres pulvérulentes et leur procédé de synthèse, définis ci-dessus, pour la décoloration des cheveux.

Un autre objet de l'invention est un procédé de décoloration des cheveux qui consiste à mélanger une composition, telle que définie ci-avant ou obtenue selon le procédé défini ci-avant, avec de l'eau oxygénée, à appliquer le mélange résultant sur les cheveux, à laisser pauser, et puis à éliminer la composition des cheveux.

Selon l'invention, on dispose ainsi de compositions de décoloration se présentant sous la forme de poudres fines mais néanmoins denses et non volatiles (absence de poussiérage). Elles sont en outre anhydres et s'écoulent bien (faible mottage; facilité de dosage accrue). Par ailleurs, leur mélange avec l'eau oxygénée est rapide et aisé, et conduit à des pâtes très homogènes. Les mélanges obtenus sont onctueux, crémeux, sans grumeau et d'aspect beaucoup plus agréable que celui obtenu avec des poudres traitées par des huiles minérales, telles que des huiles de silicone ou des huiles de paraffine, ou des cires. En outre, l'application des cataplasmes est aisée et ceux-ci adhèrent bien aux cheveux et ne glissent pas. Enfin, l'élimination de ces derniers est nettement meilleure que dans le cas des poudres à l'huile ou à la cire; les shampooings moussent, les cheveux ne sont pas gras, restent brillants et gardent un toucher naturel.

Mais d'autres caractéristiques, aspects et avantages de l'invention apparaitront encore plus clairement à la lecture de la description qui va suivre, ainsi que des divers exemples concrets, mais nullement limitatifs, destinés à l'illustrer.

Les copolymères utilisés dans le cadre de la présente invention sont des produits déja bien connus en soi et aisément synthétisables par l'homme de l'art. Ils sont par ailleurs disponibles commercialement.

Comme indiqué précédemment, ils appartiennent à la famille générales des copolymères linéaires séquencés de type polyoxyéthylène/polyoxypropylène.

De façon connue, les produits conformes à l'invention peuvent ainsi être représenter par les formules (1) ou (2) suivantes:

(AO)ₓ(EO)ₘ(AO)_{y} (1)

ou

(AO)ₓ(PO)ₘ(AO)_{y} (2)

dans lesquelles EO désigne un motif dérivant de l'oxyde d'éthylène, PO un motif dérivant de l'oxyde de propylène, AO l'un des motifs EO ou PO tels que définis ci-avant, les motifs terminaux de la chaine polymérique étant des fonctions hydroxy ou des fonctions alcoxy en C₁-C₂ (i.e. oxyméthyle ou oxyéthyle), l'un au moins de ces motifs terminaux étant nécessairement une fonction hydroxy, et les indices x, y et m étant des nombres entiers tels que ledit copolymère est, à température ambiante, liquide d'une part et soluble dans l'eau d'autre part.

Ces copolymères séquencés polyoxyéthylène/polyoxypropylène peuvent être classiquement préparés, selon les cas, (i) par réaction d'un polyoxypropylène glycol (difonctionnel) avec soit de l'oxyde d'éthylène seul (obtention dans ce cas d'un copolymère bloc linéaire de formule (EO)ₓ(PO)ₘ(EO)_{y}), soit avec un mélange oxyde d'éthylène/oxyde de propylène (obtention dans ce cas d'un copolymère statistique linéaire de formule (AO)ₓ(PO)ₘ(AO)_{y}), ou (ii) par réaction d'un polyoxyéthylène glycol (difonctionnel) avec soit de l'oxyde de propylène seul (obtention dans ce cas d'un copolymère bloc linéaire de formule (PO)ₓ(EO)ₘ(PO)_{y}), soit avec un mélange oxyde de propylène/oxyde d'éthylène (obtention dans ce cas d'un copolymère statistique linéaire de formule (AO)ₓ(EO)ₘ(AO)_{y}). Dans les cas où l'on désire mettre en oeuvre des copolymères comportant à l'une de leurs extrémités un groupement alcoxy en C₁-C₂, ceux-ci peuvent être obtenus par polyaddition séquencée et/ou statistique d'oxyde d'éthylène et d'oxyde de propylène à un alcool en C₁-C₂ (i.e. méthanol ou éthanol). Selon les proportions retenues entre les divers réactifs rentrant dans les réactions ci-dessus, on peut entre autre ajuster l'état physique (solide/liquide), la viscosité et la solubilité dans l'eau des copolymères obtenus.

De tels produits sont notamment vendus sous la dénomination commerciale générale de PLURONICS® par les Sociétés WYANDOTTE et BASF, ou sous la dénomination de SYNPERONIC® par la Société ICI.

Selon une caractéristique importante de la présente invention, on ne retient parmi les copolymères ci-dessus que ceux qui sont (i) anhydres, (ii) liquides à température ambiante et (iii) solubles dans l'eau à température ambiante. Par température ambiante, on entend ici désigner une température qui peut être comprise entre 15°C et 30°C environ. Par ailleurs, par liquide, on entend plus particulièrement désigner un produit dont la viscosité est inférieure à 1000 centipoises, de préférence inférieure à 100 centipoises. Par anhydre, on entend que la teneur en eau du produit est inférieure à 1% en poids, de préférence inférieure à 0,5% en poids. Enfin, par soluble dans l'eau, on entend plus particulièrement viser un produit polymérique dont la solubilité dans l'eau est d'au moins 1 g/l, et de préférence d'au moins 10 g/l.

Selon la présente invention, il est bien entendu possible de mettre en oeuvre un ou plusieurs des copolymères tels que ci-dessus définis.

Les copolymères préférés dans le cadre de la présente invention sont les copolymères blocs linéaires du type (EO)ₓ(PO)ₘ(EO)_{y} .

La proportion en copolymère(s) dans les compositions décolorantes selon l'invention est généralement comprise entre 5 et 27% en poids par rapport au poids total de ladite composition. De préférence, cette teneur est inférieure à 25% en poids.

Les autres constituants essentiels (dérivés peroxydés à titre d'oxydants ) ou facultatifs (régulateurs de pH, épaississants, additifs cosmétiques et autres) rentrant (ou pouvant rentrer) dans la composition des produits selon l'invention sont ceux que l'on rencontre habituellement pour des compositions décolorantes pour cheveux, notamment ceux indiqués dans la partie introductive de la présente description qui conviennent ici tout à fait.

A titre indicatif, les formulations décolorantes pour cheveux conformes à l'invention présentent généralement les compositions suivantes :
- agent(s) oxydant(s) (de préférence choisis parmi les persulfates d'alcalins) : de 20 à 60% en poids, de préférence de 30 à 50% en poids, par rapport à l'ensemble de la formulation ;
- copolymère(s): comme indiqué ci-avant ;
- régulateur(s) de pH (de préférence choisis parmi les métasilicates alcalins) : de 5 à 15% en poids, de préférence de 9 à 14% en poids, par rapport à l'ensemble de la formulation ;
- épaississant(s) : de 0,5 à 5% en poids, de préférence de 1 à 3% en poids, par rapport à l'ensemble de la composition ;
- adjuvant(s) cosmétique(s) éventuel(s): qsp 100% en poids.

Comme indiqué précédemment, les compositions décolorantes selon l'invention se présentent dans un état solide pulvérulent. Les particules constituant cette poudre ont une taille généralement inférieure à 1000 microns (1 mm) mais surtout présentent une distribution granulométrique telle que le taux pondéral des particules qui ont une taille inférieure ou égale à 65 microns (taux de fines) est remarquablement faible. Ainsi, ce taux de fines est généralement inférieur ou égal à 5% en poids, de préférence inférieur à 2% en poids, et encore plus préférentiellement inférieur à 1% en poids. Cette caractéristique fait que les poudres selon l'invention sont non poussiérantes. La taille maximale de particules indiquée ci-avant rend quant à elle les mélanges avec l'eau oxygénée beaucoup plus faciles à réaliser.

Selon un premier mode de réalisation du procédé de synthèse des compositions colorantes selon l'invention, on introduit des poudres sèches décolorantes classiques à base de dérivés peroxydés (i.e des poudres que l'on désire en particulier rendre non volatiles) dans un mélangeur mécanique (mélangeur type LÖDIGE ou WINKWORTH notamment), ces poudres sont ensuite mises en agitation, puis on incorpore dans ces dernières le ou les copolymères conformes à l'invention, et on mélange le tout, toujours par agitation mécanique, pour homogénéisation, et enfin on récupère le produit résultant qui constitue la composition selon l'invention.

Selon un autre mode de réalisation, on pulvérise simplement (par exemple par atomisation) le ou les copolymères liquides conformes à l'invention sur les poudres décolorantes initiales maintenues en agitation (agitation mécanique ou par lit fuidisé par exemple), puis on récupère le produit résultant qui constitue la composition selon l'invention.

Les deux modes de réalisation ci-dessus se font à température ambiante et sans mettre en oeuvre de tiers solvants, tels que de l'eau ou des solvants organiques (mélange à sec), même au niveau de l'étape de l'introduction du copolymère. Les proportions poudre initiale/copolymère(s) sont choisies de la même manière qu'indiqué ci-avant pour les compositions finales désirées. Dans les deux cas, les temps de mélange permettent de contrôler les granulométries finales.

Les compositions décolorantes pulvérulentes conformes à l'invention peuvent ensuite être mises en oeuvre sur les cheveux de manière classique et connue en soi dans le domaine de la décoloration capillaire. Ainsi, on mélange les compositions décolorantes sous forme de poudres avec une solution d'eau oxygénée à 6-12 % environ en volume, de préférence à 9 % environ en volume, et ceci dans un rapport généralement de l'ordre de 1 : 1, puis on mélange jusqu'à l'obtention d'une pâte homogène que l'on applique ensuite sur les cheveux et que l'on laisse poser pendant une durée comprise entre 25 et 45 minutes environ. La composition est ensuite éliminée des cheveux (rinçage à l'eau et/ou shampooings).

Des exemples concrets illustrant l'invention vont maintenant être donnés.

### EXEMPLE 1

On a préparé une composition décolorante conforme à l'invention (composition 1) qui présentait la composition suivante (% en poids) :
- - Persulfate de potassium: 25%
- - Persulfate d'ammonium: 25%
- - Métasilicate de sodium: 10%
- - Chlorure d'ammonium: 5%
- - Séquestrant: 2%
- - Silice: 13%
- - Copolymère 1^{**(1)**}: 20%

^{**(1)**} : copolymère de type 8OE/30OP/8OE vendu sous le nom de SYNPERONIC PE/L62 par la Société ICI.

Le mode opératoire était le suivant : on a introduit dans un mélangeur de type LÖDIGE les différents composés solides constituant la poudre décolorante initiale (i.e ne contenant pas le copolymère), on les a mélangés à sec pendant 20 minutes dans le mélangeur, puis on a introduit dans ce dernier le copolymère servant d'agent d'enrobage, et on a mélangé le tout (toujours à sec) pour homogénéisation pendant 20 minutes.

La granulométrie de la poudre décolorante finale obtenue (déterminée par tamissage) est indiquée dans le tableau donné ci-après (composition 1). Ce tableau indique le % en poids des particules présentes dans la composition dont la taille est comprise dans un intervalle donné de taille.

A titre comparatif, on a également indiqué dans ce tableau la granulométrie de la poudre décolorante avant l'introduction du copolymère (composition 0).

### EXEMPLE 2

En reprenant le mode opératoire décrit à l'exemple 1, on a préparé une deuxième composition conforme à l'invention (composition 2) semblable à celle de l'exemple 1 à cette seule différence près que le copolymère 1 a été remplacé ici par un copolymère de type 6OE/19OP/6OE vendu sous le nom commercial de PLURONIC PE 4300 par la Société BASF.

La granulométrie de la poudre décolorante finale obtenue (déterminée par tamissage) est indiquée dans le tableau donné ci-après (composition 2).

### EXEMPLE 3

En reprenant le mode opératoire de l'exemple 1, on a préparé une troisième composition conforme à l'invention (composition 3) présentant cette fois la composition pondérale suivante :
- - Persulfate de potassium: 60%
- - Persulfate d'ammonium: 20%
- - Métasilicate de sodium: 10%
- - Chlorure d'ammonium: 5%
- - Copolymère 1^{**(1)**}: 5%

^{**(1)**} : copolymère de type 8OE/30OP/8OE vendu sous le nom de SYNPERONIC PE/L62 par la Société ICI.

La granulométrie de la poudre décolorante finale obtenue (déterminée par tamissage) est indiquée dans le tableau donné ci-après (composition 3).

La granulométrie du mélange de poudres avant l'introduction du copolymère était identique à celle de la composition 0 de l'exemple 1.

Les trois compositions 1, 2 et 3 étaient dépourvues de toute poussière.

**TABLEAU**

| Taille Φ (µm) | Composition 0 | Composition 1 | Composition 2 | Composition 3 |
|---|---|---|---|---|
| Φ>710 | 2,2 | 30,24 | 29,02 | 32,00 |
| 355< Φ <710 | 7,8 | 31,64 | 27,96 | 50,60 |
| 100< Φ 355 | 28,46 | 21,56 | 21,60 | 13,11 |
| 63< Φ <100 | 52,86 | 15,94 | 19,68 | 3,26 |
| 0< Φ <63 | 7,74 | 0,20 | 0,40 | 0,00 |

## Revendications

1. Compositions cosmétiques anhydres pulvérulentes pour la décoloration des cheveux, à base de dérivés peroxydés comme agents oxydants, caractérisées par le fait qu'elles comprennent en outre au moins un copolymère linéaire séquencé bloc et/ou statistique de type polyoxyéthylène/polyoxypropylène qui est anhydre et qui, à température ambiante, est liquide d'une part et soluble dans l'eau d'autre part.

2. Compositions selon la revendication 1, caractérisées en ce que ledit copolymère répond à la formule (1) ou (2) suivante :
(AO)ₓ(EO)ₘ(AO)_{y} (1)
ou
(AO)ₓ(PO)ₘ(AO)_{y} (2)
dans lesquelles EO désigne un motif dérivant de l'oxyde d'éthylène, PO un motif dérivant de l'oxyde de propylène, AO l'un des motifs EO ou PO tels que définis ci-avant, les motifs terminaux de la chaine polymérique étant des fonctions hydroxy ou des fonctions alcoxy en C₁-C₂, l'un au moins de ces motifs terminaux étant nécessairement une fonction hydroxy, et les indices x, y et m étant des nombres entiers tels que ledit copolymère est, à température ambiante, liquide d'une part et soluble dans l'eau d'autre part.

3. Compositions selon la revendication 2, caractérisées en ce que ledit copolymère est un copolymère du type (EO)ₓ(PO)ₘ(EO)_{y}.

4. Compositions selon l'une quelconque des revendications précédentes, caractérisées en ce que la teneur en copolymère(s) est comprise entre 5 et 27% en poids par rapport au poids total de la composition.

5. Compositions selon la revendication 4, caractérisées en ce que ladite teneur est inférieure à 25% en poids.

6. Compositions selon l'une quelconque des revendications précédentes, caractérisées en ce que la teneur en agents oxydants de type dérivés péroxydés est comprise entre 20 et 60% en poids, de préférence de 30 à 50% en poids, par rapport à l'ensemble de la formulation.

7. Compositions selon la revendication 6, caractérisées en ce que les dérivés péroxydés sont choisis parmi des persulfates d'alcalins.

8. Compositions selon l'une quelconque des revendications précédentes, caractérisées en ce que le taux pondéral des particules les constituant qui ont une taille inférieure ou égale à 65 microns est inférieur ou égal à 5% en poids, de préférence inférieur à 2% en poids, et encore plus préférentiellement inférieur à 1% en poids.

9. Compositions selon l'une quelconque des revendications précédentes, caractérisées par le fait que la taille des particules les constituant ont une taille inférieure à 1000 microns (1 mm).

10. Procédé de préparation d'une composition telle que définie à l'une quelconque des revendications précédentes, caractérisé en ce qu'il consiste à mélanger, en milieu non solvant et à température ambiante, une poudre sèche décolorante à base de dérivés peroxydés solides, avec un copolymère tel que défini dans les revendications précédentes.

11. Utilisation d'une composition telle que définie à l'une quelconque des revendications 1 à 9 ou obtenue selon le procédé de la revendication 10 pour la décoloration des cheveux.

12. Procédé de décoloration des cheveux, caractérisé par le fait qu'il consiste à mélanger une composition telle que définie à l'une quelconque des revendications 1 à 9 ou obtenue selon le procédé de la revendication 10 avec de l'eau oxygénée, à appliquer le mélange résultant sur des cheveux, à laisser pauser, et enfin à éliminer la composition des cheveux.

## Claims

1. Pulverulent anhydrous cosmetic compositions for bleaching hair, based on peroxidized derivatives as oxidizing agents, characterized by the fact that they comprise, in addition, at least one block and/or random linear copolymer of the polyoxyethylene/polyoxypropylene type which is anhydrous and which, at room temperature, is liquid on the one hand and soluble in water on the other.

2. Compositions according to Claim 1, characterized in that the said copolymer corresponds to the following formula (1) or (2):
(AO)ₓ(EO)ₘ(AO)_{y} (1)
or
(AO)ₓ(PO)ₘ(AO)_{y} (2)
in which EO designates a unit which is derived from ethylene oxide, PO a unit which is derived from propylene oxide, AO one of the EO or PO units as defined above, the terminal units of the polymeric chain being hydroxyl functional groups or C₁-C₂ alkoxy functional groups, at least one of these terminal units being necessarily a hydroxyl functional group, and the subscripts x, y and m being integers such that the said copolymer is, at room temperature, liquid on the one hand and soluble in water on the other.

3. Compositions according to Claim 2, characterized in that the said copolymer is a copolymer of the (EO)ₓ(PO)ₘ(EO)_{y} type.

4. Compositions according to any one of the preceding claims, characterized in that the content of copolymer(s) is between 5 and 27% by weight relative to the total weight of the composition.

5. Compositions according to Claim 4, characterized in that the said content is less than 25% by weight.

6. Compositions according to any one of the preceding claims, characterized in that the content of oxidizing agents of the peroxidized derivative type is between 20 and 60% by weight, preferably 30 to 50% by weight, relative to the whole formulation.

7. Compositions according to Claim 6, characterized in that the peroxidized derivatives are chosen from alkali metal persulphates.

8. Compositions according to any one of the preceding claims, characterized in that the quantity by weight of the particles constituting them which have a size less than or equal to 65 microns is less than or equal to 5% by weight, preferably less than 2% by weight, and still more preferably less than 1% by weight.

9. Compositions according to any one of the preceding claims, characterized by the fact that the size of the particles constituting them have a size less than 1000 microns (1 mm).

10. Process for preparing a composition as defined in any one of the preceding claims, characterized in that it consists in mixing, in non-solvent medium and at room temperature, a dry bleaching powder based on solid peroxidized derivatives, with a copolymer as defined in the preceding claims.

11. Use of a composition as defined in any one of Claims 1 to 9 or which is obtained according to the process of Claim 10 for bleaching the hair.

12. Process for bleaching the hair, characterized by the fact that it consists in mixing a composition as defined in any one of Claims 1 to 9 or which is obtained according to the process of Claim 10, with hydrogen peroxide, in applying the resulting mixture to the hair, in allowing to act, and finally in removing the composition from the hair.

## Patentansprüche

1. Pulverförmige, wasserfreie kosmetische Zusammensetzungen für die Entfärbung von Haaren auf der Basis von Peroxidderivaten als Oxidationsmittel, dadurch gekennzeichnet, daß sie außerdem mindestens ein sequentielles lineares Block-Copolymer und/oder sequentielles lineares statistisches Copolymer vom Polyoxyethylen/Polyoxypropylen-Typ enthalten, das wasserfrei ist und bei Umgebungstemperatur einerseits flüssig und andererseits in Wasser löslich ist.

2. Zusammensetzungen nach Anspruch 1, dadurch gekennzeichnet, daß das obengenannte Copolymer die folgende Formel (1) oder (2)
(AO)ₓ(EO)ₘ(AO)_{y} (1)
oder
(AO)ₓ(PO)ₘ(AO)_{y} (2)
aufweist, worin bedeuten:
- EO eine Einheit, die von Ethylenoxid abgeleitet ist,
- PO eine Einheit, die von Propylenoxid abgeleitet ist,
- AO eine EO-Einheit oder eine PO-Einheit, die wie zuvor definiert sind,
wobei die endständigen Einheiten der Polymerkette Hydroxygruppen oder C₁₋₂-Alkoxygruppen sind und mindestens eine dieser endständigen Einheiten notwendigerweise eine Hydroxygruppe ist und wobei die Indizes x, y und m solche ganzen Zahlen sind, das das Copolymer bei Umgebungstemperatur einerseits flüssig und andererseits in Wasser löslich ist.

3. Zusammensetzungen nach Anspruch 2, dadurch gekennzeichnet, daß das Copoylmer ein (EO)ₓ(PO)ₘ(EO)_{y}-Copolymer ist.

4. Zusammensetzungen nach einem der vorhergehenden Ansprüche, dadurch gekennzeichnet, daß der Gehalt an dem Copolymer oder den Copolymeren 5 bis 27 Gew.-%, bezogen auf das Gesamtgewicht der Zusammensetzung, beträgt.

5. Zusammensetzungen nach Anspruch 4, dadurch gekennzeichnet, daß der obengenannte Gehalt kleiner als 25 Gew.-% ist.

6. Zusammensetzungen nach einem der vorhergehenden Ansprüche, dadurch gekennzeichnet, daß der Gehalt an Oxidationsmitteln vom Typ der Peroxidderivate 20 bis 60 Gew.-%, vorzugsweise 30 bis 50 Gew.-%, bezogen auf die gesamte Formulierung, beträgt.

7. Zusammensetzungen nach Anspruch 6, dadurch gekennzeichnet, daß die Peroxidderivate unter Alkalipersulfaten ausgewählt sind.

8. Zusammensetzungen nach einem der vorhergehenden Ansprüche, dadurch gekennzeichnet, daß von den Partikeln, aus denen diese Zusammensetzungen bestehen, ein gewichtsbezogenener Anteil, der kleiner als oder gleich 5 Gew.-%, vorzugsweise kleiner als 2 Gew.-% und noch bevorzugter kleiner als 1 Gew.-% ist, eine Größe kleiner als oder gleich 65 µm aufweist.

9. Zusammensetzungen nach einem der vorhergehenden Ansprüche, dadurch gekennzeichnet, daß die Partikel, aus denen diese Zusammensetzungen bestehen, kleiner als 1000 µm (1 mm) sind.

10. Verfahren zur Herstellung einer wie in einem der vorhergehenden Ansprüche definierten Zusammensetzung, dadurch gekennzeichnet, daß in einem lösungsmittelfreien Medium und bei Umgebungstemperatur ein trockenes, entfärbendes Pulver auf der Basis fester Peroxidderivate mit einem wie in den vorhergehenden Ansprüchen definierten Copolymer vermischt wird.

11. Verwendung einer wie in einem der Ansprüche 1 bis 9 definierten oder nach dem Verfahren von Anspruch 10 hergestellten Zusammensetzung für die Entfärbung von Haaren.

12. Verfahren zur Entfärbung von Haaren, das durch folgende Schritte gekennzeichnet ist:
- Vermischen einer wie in einem der Ansprüche 1 bis 9 definierten oder nach dem Verfahren von Anspruch 10 hergestellten Zusammensetzung mit Wasserstoffperoxid,
- Aufbringen des resultierenden Gemischs auf die Haare,
- Einwirkenlassen des Gemisch und schließlich
- Entfernen der Zusammensetzung aus den Haaren.
